# DEMANDE DE BREVET EUROPEEN

(11) **EP 0 826 340 A2**
(43) Date de publication de la demande: **04.03.1998**
(21) Numéro de dépôt: 97402035.6
(22) Date de dépôt: 01.09.1997
(51) Int. Cl.: A61B 17/068, A61B 17/064, A61B 17/88

(54) **Support d'agrafe chirurgicale du type élastique, superélastique ou à mémoire de forme**

(30) Priorité: 03.09.1996 FR 9610730
(71) Demandeur: MEDINOV-AMP, 42300 Roanne (FR); Barouk, Louis Samuel, F-33370 Yvrac (FR)
(72) Inventeur: Barouk, Louis Samuel, 33370 Yvrac (FR); Caubit, Frédéric, 74000 Annecy (FR)
(74) Mandataire: Martin, Jean-Paul

(57) **Abrégé**

Support (50) d'agrafe chirurgicale (5) d'ostéosynthèse constituée de deux branches ou pattes (6) d'ancrage osseux, reliées par une partie centrale (7), les branches d'ancrage pouvant après sollicitation forcée reprendre automatiquement leur position initiale par un effet élastique, ou superélastique, ou à mémoire de forme du matériau; ce support comporte des moyens mécaniques (51) adaptés pour maintenir écartées l'une de l'autre les pattes d'ancrage dans leur position d'insertion chirurgicale, et pouvant exercer une pression d'écartement sur lesdites pattes ; ceci assure ainsi sur une zone de fracture osseuse une pression permanente des deux parties osseuses l'une contre l'autre. Ce support peut être constitué par exemple par une paire de mâchoires (51) dans chacune desquelles est formée une empreinte (52) de réception de la partie centrale d'agrafe (5), et par une pince (53) de manoeuvre des mâchoires (51).

## Description

La présente invention a pour objet un support d'agrafe chirurgicale, cette agrafe comportant deux branches ou pattes d'ancrage osseux, reliées par une partie centrale.

Ce support a pour but de mettre à profit les caractéristiques particulière de ce type d'agrafe, à savoir, soit des caractéristiques élastiques du type classique, soit des caractéristiques dites superélastiques ou à mémoire de forme, constituées en des matériaux respectifs différents. Les agrafes superélastiques présentent, par rapport aux agrafes élastiques traditionnelles, la possibilité d'une déformation réversible très supérieure.

Les agrafes du type dit à mémoire de forme sont de plus en plus utilisées dans le domaine chirurgical, notamment pour la réduction de certaines fractures osseuses, par exemple pour des os du pied. L'originalité des matériaux à mémoire de forme (AMF), constitués par certains alliages tels que le nickel-titane et le Cu-Zn-Al, réside dans le fait que de part et d'autre d'une température de transition, ils présentent une structure cristalline austénitique stable à chaud et une structure martensitique stable à froid. La température de transition, donc de passage d'une structure à l'autre, peut varier en fonction de divers paramètres. L'expression d'alliage à mémoire de forme provient du fait qu'une pièce qui a été conformée dans sa structure austénitique, puis a subi un passage en structure martensitique par refroidissement au-dessous de la structure de transition, peut se voir imposer une forme seconde modifiée, mais retrouve sa forme première par retour à la structure austénitique grâce à une modification appropriée de sa température.

Ainsi, avec les alliages à mémoire, en fonction du traitement thermomécanique qui leur est imposé, il est possible de réaliser des agrafes soit à effet superélastique, soit à effet caoutchoutique, à effet amortissant, à effet mémoire simple sens ou encore à effet mémoire double sens.

La partie centrale de l'agrafe peut présenter une forme rectiligne simple, par exemple dans le cas où pour l'usage que doit en faire le chirurgien, celui-ci ne souhaite pas une réduction significative de sa longueur, qui entraînerait une compression trop importante de l'agrafe.

Dans d'autres cas, la partie centrale de l'agrafe peut présenter une forme ondulée en S ou en W dans le plan vertical ou dans le plan horizontal, ou bien encore une forme en boutonnière constituée de deux languettes séparées l'une de l'autre, mais dont les extrémités se rejoignent au niveau des extrémités opposées de la partie centrale de l'agrafe.

A basse température, c'est-à-dire au-dessous de la température de transition entre l'état martensitique et l'état austénitique, l'alliage constituant une agrafe à mémoire de forme, est malléable. De ce fait, ses branches peuvent être placées en position écartée l'une de l'autre et sa partie centrale peut être rectiligne, ou en forme d'oeillet aplati. Lorsque l'agrafe est posée sur le patient, sa température remonte jusqu'à celle du corps du patient, soit environ 37°C. Cette augmentation de température entraîne un passage de la structure martensitique à la structure austénitique, et corrélativement une déformation de la partie centrale et un rapprochement des branches d'ancrage, qui maintiennent les éléments osseux appliqués l'un contre l'autre.

L'alliage peut donc avoir une température de fin de structure austénitique inférieure à 37°C, auquel cas le chirurgien doit refroidir l'agrafe avant sa mise en place pour qu'elle soit en état martensitique.

L'alliage peut aussi avoir une température de transition ( fin de la structure austénitique ) supérieure à 37°C. Dans ce cas, l'implant est livré au chirurgien en position de pose sur le patient, c'est-à-dire en état martensitique. Le chirurgien chauffe alors l'implant après sa mise en place, pour le faire passer à l'état austénitique.

On connaît une réalisation d'agrafe chirurgicale dont la partie centrale peut être rectiligne à une température inférieure à la température de transformation d'une phase à l'autre, et ondulée à une température supérieure à cette température de transformation.

Jusqu'à présent, on a conservé de telles agrafes de manière stérile dans des enceintes froides d'où le chirurgien les extrait juste avant de les poser sur les patients. Ceci implique que l'enceinte froide de conservation doit être disponible à proximité immédiate du bloc chirurgical. Mais il se présente alors le risque que pour une raison fortuite, le chirurgien ne puisse immédiatement poser l'agrafe, qui de ce fait se réchauffe et change de forme avant d'avoir pu être installée, ce qui la rend inutilisable.

Pour surmonter cet inconvénient, on a proposé de conserver les agrafes dans des dispositifs de conditionnement permettant la mise en oeuvre de l'agrafe pendant un certain temps après que ce conditionnement ait été extrait de son emplacement de conservation. Ainsi, un conditionnement contenant un produit cryogénique permet de maintenir l'agrafe dans sa géométrie appropriée à la pose pendant une certaine durée après son extraction du dispositif de conditionnement, grâce au fait que les branches sont maintenues en contact avec le fluide froid de conservation.

Toutefois, un tel conditionnement exige un produit cryogénique. En outre, le chirurgien doit extraire manuellement l'agrafe avant de procéder à sa pose, ce qui peut présenter des difficultés.

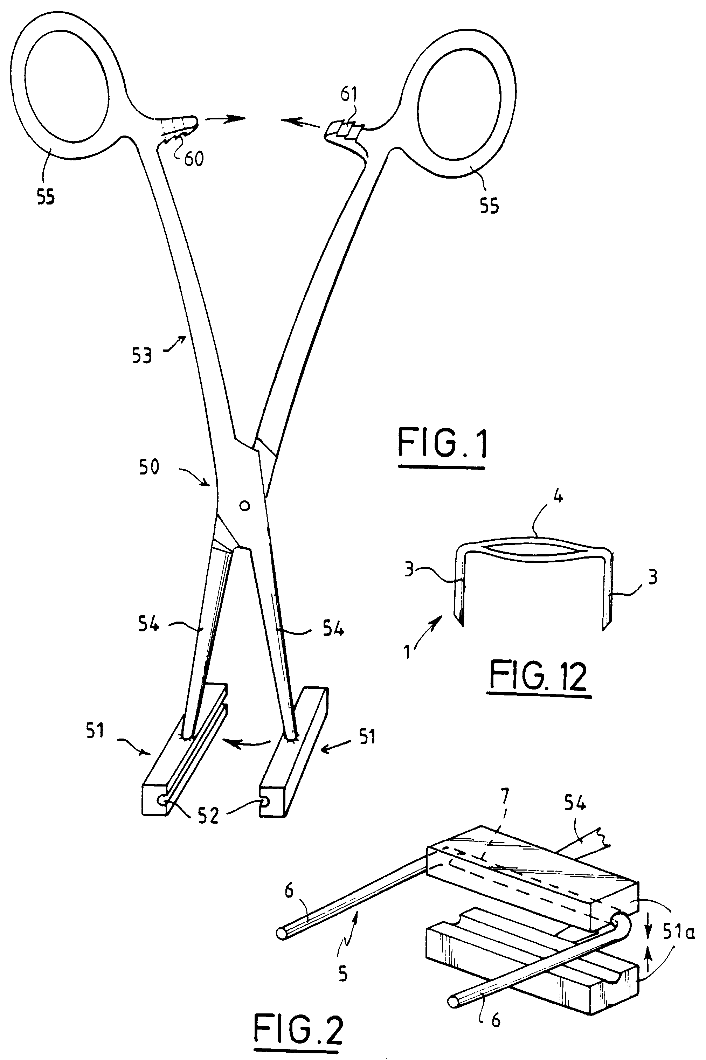

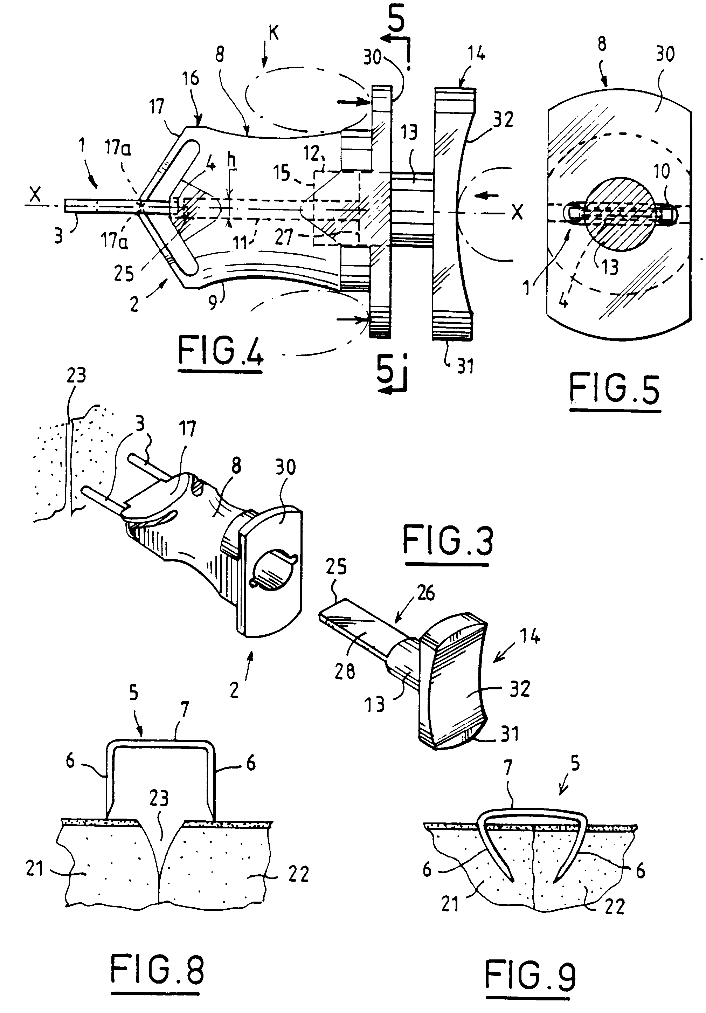

Au titre de l'état de la technique, on peut citer les documents suivants :
- le WO-83/00615 décrit une agrafeuse-dégrafeuse comportant des tiges mobiles transversalement dans le plan de l'agrafe, de manière à en écarter les pattes avant la pose, puis à libérer celles-ci, et ce par commande manuelle.
- le US-A-3 283 557 concerne un ôte-agrafe chirurgical pourvu d'une pince qui peut prendre appui sous la partie centrale de l'agrafe. Cette pince peut exercer une pression permettant d'écarter les pattes l'une de l'autre et ainsi de libérer l'agrafe.
- le US-A-4 217 902 décrit un écarteur manuel des pattes d'une agrafe chirurgicale.
- le DE-A-3 335 986 a trait à un appareil de pose d'agrafes permettant d'écarter les pattes de l'agrafe lorsqu'elle sort de l'appareil, par pression sur sa partie centrale qui subit une flexion (Fig.2 et 3).

Tous ces dispositifs ont en commun le fait de n'agir que momentanément, par commande manuelle sur l'élasticité et l'écartement des pattes. Ils n'assurent donc aucun maintien de cet écartement de façon permanente par des moyens autonomes, qui rendraient l'appareil transportable avec l'agrafe ayant ses pattes maintenues écartées.

L'invention a un triple but : s'affranchir de la nécessité de disposer d'un produit cryogénique, rendre plus aisée l'opération de pose de l'agrafe par le chirurgien, et réaliser un support d'agrafe permettant de maintenir les pattes de celle-ci écartées de façon permanente sans intervention manuelle, rendant le support et son agrafe autonomes dans cet état, et aisément transportables.

Le support d'agrafe chirurgicale d'ostéosynthèse visé par l'invention est destiné à une agrafe des types mentionnés ci-dessus, dont les branches, pattes d'ancrage ou partie centrale peuvent après sollicitation forcée reprendre automatiquement leur position initiale par un effet élastique, ou superélastique, ou à mémoire de forme du matériau.

Conformément à l'invention, ce support comporte des moyens mécaniques autonomes adaptés pour maintenir écartées l'une de l'autre les pattes d'ancrage en les bloquant dans leur position d'insertion, tandis que la partie centrale de l'agrafe est maintenue avec un allongement maximum; ces moyens mécaniques peuvent exercer une pression d'écartement qui permet le retour des pattes vers leur position initiale, pour assurer ainsi sur une zone de fracture osseuse, une pression permanente des deux parties osseuses l'une contre l'autre. Ainsi est facilitée la soudure osseuse des deux éléments osseux mécaniquement reliés par l'agrafe.

Un tel support est autonome et aisément transportable avec les pattes de son agrafe maintenues en permanence écartées par le système de blocage mécanique.

Selon le mode de réalisation souhaité, le support comporte des mâchoires de préhension dont la longueur est au moins légèrement supérieure à la longueur de la partie centrale de l'agrafe lorsque ladite partie centrale est rectiligne, ou supérieure à la longueur totale déployée de ladite partie centrale lorsque celle-ci présente une ondulation ou toute autre forme autorisant un allongement, telle qu'un oeillet ou boutonnière.

L'ensemble du support et de l'agrafe chirurgicale peut être présenté sous forme d'une unité stérile, directement prête à l'utilisation par le chirurgien, sans nécessiter la mise en oeuvre d'un produit cryogénique, grâce à des moyens uniquement mécaniques.

L'invention permet donc de simplifier la mise en place de l'agrafe en s'affranchissant des étapes, nécessaires jusqu'à présent, d'un refroidissement ou d'un chauffage en bloc opératoire à l'aide d'un gabarit de maintien. En outre, le support sert également d'outil de pose de l'agrafe, facilitant cette pose au chirurgien.

Suivant un second mode de réalisation de l'invention, lesdits moyens mécaniques sont conformés en outre pour pouvoir se refermer sur la partie centrale de l'agrafe lorsque cette partie est sous une forme géométrique adaptée à sa pose chirurgicale.

Dans ce cas, l'agrafe est maintenue dans sa conformation adaptée à la pose chirurgicale à la fois dans sa partie centrale et par ses pattes d'ancrage orientées suivant un écartement approprié.

Suivant un mode de réalisation particulier de l'invention, lesdits moyens comprennent deux éléments allongés formant mâchoires, dans chacun desquels est ménagée une empreinte conjuguée de la forme de la partie centrale de l'agrafe afin que ladite partie centrale puisse être emprisonnée par les deux mâchoires appliquées l'une sur l'autre avec leurs empreintes en vis-à-vis; les extrémités de ces mâchoires sont conformées pour former des butées de retenue des pattes en position d'écartement, et le support comprend en outre une pince d'écartement ou de rapprochement des mâchoires, aux branches de laquelle ces dernières sont fixées.

Dans ce mode de réalisation, si la partie centrale de l'agrafe est rectiligne, la longueur utile des mâchoires est au moins légèrement supérieure à la longueur de la partie centrale. Si la partie centrale de l'agrafe est conformée pour pouvoir s'allonger, par exemple à l'aide d'une ondulation déformable en forme de S horizontal, ou bien encore de toute autre forme ou disposition autorisant un allongement, telle qu'un oeillet (ou boutonnière), la longueur des mâchoires est au moins égale à la longueur déployée, donc maximale de la partie centrale lorsqu'elle est aplatie.

Suivant un autre mode de réalisation de l'invention, les moyens mécaniques de support comprennent un corps présentant à une extrémité un logement de réception de la partie centrale l'agrafe et des volets flexibles de retenue des branches de l'agrafe en position écartée, et des moyens sont prévus pour extraire l'agrafe du corps et pour la poser.

Suivant une caractéristique de ce mode de réalisation, les moyens d'extraction de l'agrafe consistent en un piston monté coulissant dans l'évidement longitudinal de manière que son extrémité puisse exercer une poussée longitudinale sur la partie centrale de l'agrafe.

Le support se présente alors sous une forme comparable à celle d'une seringue.

Après la mise en place de l'agrafe sur le support, qui forme à la fois gabarit et outil de pose, c'est-à-dire pendant les étapes de conditionnement (stérilisation-expédition-stockage), la température de l'agrafe peut augmenter progressivement dans certaines limites (+50°C -10°C maximum), et donc dépasser la température de début de passage à la phase austénitique, s'il s'agit d'une agrafe en alliage de mémoire de forme.

A ce moment l'agrafe tend à commencer à changer de forme. Etant donné qu'elle est bloquée sur le support, qui empêche le rapprochement de branches de l'agrafe et le cas échéant exerce en outre sur sa partie centrale une contention s'opposant à sa déformation, cet empêchement à changer de forme engendre des contraintes internes et de ce fait une élévation de la température du début de passage à la phase austénitique. La température correspondante augmentant, il en résulte une baisse des contraintes, et un bouclage automatique, qui évite toute détérioration de l'agrafe.

Lorsque le chirurgien libère l'agrafe à la mise en place, il libère également les contraintes internes. Si l'agrafe est en matériau élastique, les pattes reviennent à leur position initiale prédéterminée. Si l'agrafe est à mémoire de forme, les températures de transition de l'agrafe évoluent, et l'agrafe peut passer progressivement à l'état austénitique et changer de forme par rapprochement de ses branches et éventuellement modification de la conformation de sa partie centrale.

L'insertion de la partie centrale de l'agrafe dans un logement de géométrie appropriée du support oblige cette partie centrale à s'appuyer plus ou moins fortement sur les parois du logement, car la partie centrale tend à se déformer. De ce fait le logement exerce une fonction de retenue en place de l'agrafe, complémentaire du maintien par les volets flexibles.

Dans le cas où la partie centrale à une forme en oeillet, celui-ci tend à se dilater en s'appliquant contre les parois du logement, ce qui contribue à maintenir l'agrafe en place jusqu'à sa pose.

Toutefois cette retenue de l'agrafe par sa partie centrale n'est pas indispensable, dès lors que les pattes sont maintenues écartées.

D'autres particularités et avantages de l'invention apparaîtront au cours de la description qui va suivre, faite en référence aux dessins annexés qui en illustrent plusieurs formes de réalisation à titre d'exemples non limitatifs.
- la Fig.1 est une vue en perspective d'une première forme de réalisation du support d'agrafe chirurgicale, comprenant une pince aux extrémités de laquelle sont fixés des éléments allongés adaptés à coopérer avec une agrafe, la pince étant représentée ouverte;
- la Fig.2 est une vue en perpective partielle du support de la figure 1 montrant ses éléments constitutifs en position écartée l'une de l'autre et la partie centrale d'une agrafe engagée dans l'un de ces éléments;
- la Fig.3 est une vue en perspective éclatée d'une seconde forme de réalisation du support d'agrafe chirurgicale conforme à l'invention;
- la Fig.4 est une vue en élévation longitudinale à échelle agrandie du support d'agrafe de la figure 3;
- la Fig.5 est une vue en coupe transversale et élévation suivant 5-5 de la figure 4;
- la Fig.6 est une vue en élévation longitudinale du support d'agrafe suivant la flèche K de la figure 4;
- la Fig.7 est une vue en élévation et coupe longitudinale partielle du support d'agrafe de la figure 6;
- la Fig.8 est une vue en élévation partielle montrant une agrafe chirurgicale en position d'utilisation avec ses branches écartées avant enfoncement dans des éléments osseux à fixer;
- la Fig.9 est une vue analogue à la figure 8, montrant l'agrafe avec ses branches d'ancrage enfoncées dans les éléments osseux et rapprochées l'une de l'autre;
- la Fig.10 est une vue en élévation latérale d'une troisième forme de réalisation du support d'agrafe chirurgicale visée par l'invention;
- la Fig.11 est une vue de dessus du support d'agrafe de la figure 10;
- le Fig.12 est une vue en perspective d'une agrafe à mémoire de forme et à partie centrale en forme d'oeillet.

Le support d'agrafe chirurgicale 50 illustré à la figure 1 est destiné à permettre la pose d'une agrafe chirurgicale 5 (Fig.2,8,9) constituée de deux branches 6 ou pattes d'ancrage osseux et d'une partie centrale pouvant être par exemple rectiligne comme la partie centrale 7.

L'agrafe peut revêtir des géométries différentes, notamment dans sa partie centrale, qui peut être un oeillet 4 (Fig.4-5 et 12). Son matériau constitutif peut être soit élastique, soit superélastique, soit encore en alliage à mémoire de forme.

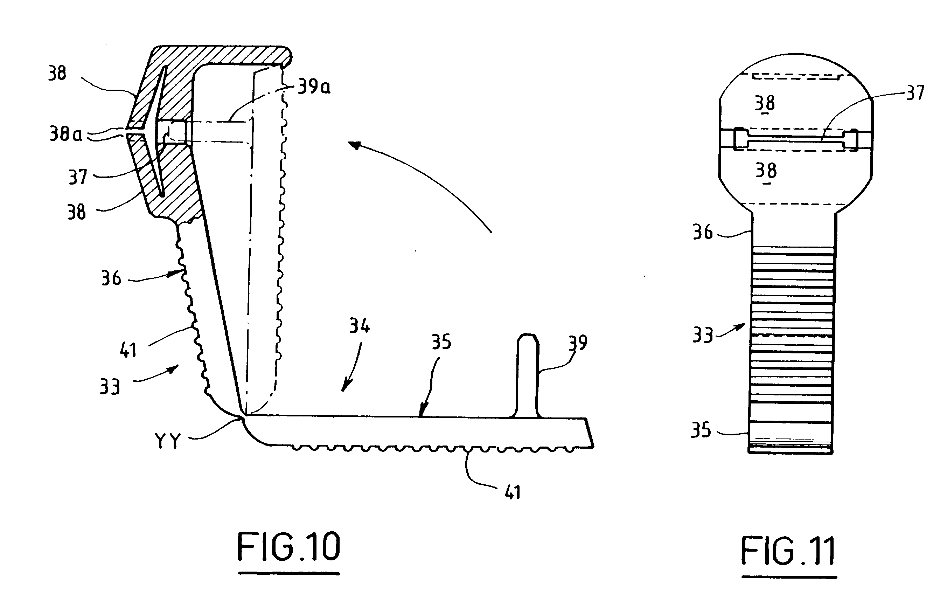

Le support 50 comprend des moyens mécaniques adaptés pour maintenir écartées l'une de l'autre, les pattes 6 dans leur position de pose chirurgicale (Fig.8), dans laquelle elles sont à peu près parallèles.

Dans l'exemple de réalisation illustré aux figures 1 et 2, ces moyens mécaniques comprennent deux éléments allongés 51 formant mâchoires, dans chacun desquels est ménagée une empreinte longitudinale 52. Cette dernière est conjuguée de la forme de la partie centrale 7 de l'agrafe 5, et peut revêtir, par exemple comme visible aux figures 1 et 2, la forme d'une rainure de section hémisphérique ou en U. Ainsi, la partie centrale 7 peut être emprisonnée par les deux mâchoires 51 lorsque celles-ci sont appliquées l'une sur l'autre avec leurs empreintes 52 en vis-à-vis.

A cet effet, le support 50 comporte une pince 53 dont les branches 54 ont leurs extrémités fixées à une mâchoire respective 51 par tout moyen approprié connu en soi. Les extrémités opposées des branches 54 peuvent comporter des boucles 55 permettant la prise en main du support pour la manutention de l'ensemble pince-agrafe lors de la stérilisation ou lors de la pose de ladite agrafe par le chirurgien.

D'autre part, les extrémités opposées des mâchoires 51 sont conformées pour pouvoir former des butées de retenue des pattes ou branches 6 en position d'écartement, lorsque ces pattes 3 sont convenablement orientées par rapport aux mâchoires 51. Concrètement, cette orientation est celle dans laquelle les pattes 6 s'étendent latéralement aux faces d'extrémité 51a et près de celles-ci (Fig.2) de telle sorte qu'elles viennent par effet élastique, surélastique ou mémoire de forme, s'appliquer sur ces faces terminales 51a lorsque l'agrafe 5 est mise en place et les mâchoires 51 refermées sur la partie centrale 7. En effet, ces faces 51a en contact avec une portion de la longueur des pattes 3 font obstacle à un rapprochement de celles-ci.

Les extrémités des branches 54 de la pince 53 sont munies de crans 60,61 pouvant engrener ensemble lorsque la pince 53 est refermée et les mâchoires 51 appliquées l'une sur l'autre. Ainsi, le support 50 peut être transporté de manière autonome, avec les pattes 6 de l'agrafe 5 maintenues en permanence écartées l'une de l'autre par le système de blocage formé par les crans 60 et 61 agencés près des boucles 55.

La longueur des mâchoires 51 est au moins légèrement supérieure à la partie centrale de l'agrafe si cette partie centrale est rectiligne (agrafe 5), ou au moins égale à la longueur déployée maximum de la partie centrale si celle-ci a une forme telle qu'elle peut s'allonger, comme pour l'oeillet 4 de l'agrafe 1.

La mise en oeuvre du support 50 s'effectue de manière très simple : la pince 53 étant ouverte et par conséquent les mâchoires 51 étant écartées l'une de l'autre, l'opérateur ou le chirurgien introduit dans l'une des empreintes 52 la partie centrale 7 de l'agrafe dans sa position de pose chirurgicale avec ses pattes 6 écartées et à peu près parallèles. Puis il referme la pince 53 de manière à emprisonner la partie centrale 7 et si nécessaire à écraser celle-ci dans les empreintes complémentaires 52, comme illustré par les flèches sur la figure 2. Après stérilisation de l'ensemble, l'agrafe 5 peut alors être posée sur la fracture 23 de la zone osseuse (Fig.3,8,9), car elle est maintenue dans la géométrie appropriée par les mâchoires 51 refermées, dont les faces terminales 51a s'opposent à tout rapprochement des pattes 3.

Le support 50 constitue à la fois un porte-agrafe et un outil de pose de l'agrafe 5 par le chirurgien.

L'avantage essentiel de ce support est d'être simple dans sa réalisation et dans son utilisation. En outre, ce support est réutilisable pour la pose d'autres agrafes. Cette dernière possibilité permet alors d'utiliser à nouveau un outil spécifique non jetable, ce qui peut être un critère de choix déterminant pour le chirurgien.

Dans le second mode de réalisation illustré aux figures 3 à 7, le support comprend un corps 8 ayant une forme sensiblement tubulaire, pouvant présenter une légère concavité périphérique 9 concentrique à l'axe longitudinal XX du corps tubulaire 8. Dans ce dernier est ménagé longitudinalement un évidement constitué, dans l'exemple décrit, en deux parties décalées longitudinalement 11 et 12.

La chambre 12 s'étend depuis la face du corps 8 opposée à son extrémité destinée à recevoir l'agrafe 1 ou 5. Dans cette chambre peut coulisser longitudinalement une partie 13 de la tige 26 d'un piston 14 jusqu'à ce que cette partie 13 vienne en butée contre un épaulement transversal 15 constituant le fond de la chambre 12.

Cette dernière se prolonge axialement par une fente 11 dont une extrémité 10 constitue le logement de la partie centrale 4 (ou 7) de l'agrafe 1 (ou 5). De part et d'autre du logement 10 s'articulent sur le nez 16 deux volets flexibles 17, reliés à leur base au corps 8, disposés en V et dont les bords libres 17a se font face au niveau du logement 10 et des branches 3 (ou 6) de l'agrafe 1 (ou 5).

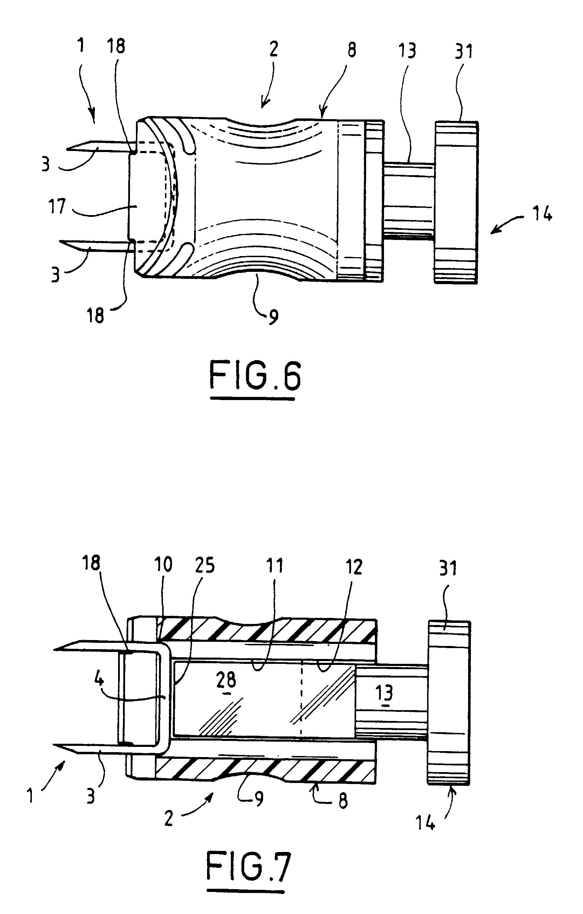

Des crans 18 (figures 6 et 7) sont agencés sur les extrémités des volets 17 et tournés vers l'extérieur, de manière à recevoir chacun en appui une branche 3 (ou 6), et à constituer pour celle-ci et donc pour l'agrafe des butées de retenue et de maintien des branches 3 en position écartée (figures 4, 5, 6) c'est-à-dire de pose de l'agrafe.

Cet agencement des volets 17 avec leurs crans 18 de blocage des branches de l'agrafe permet de maintenir celles-ci en position écartée, de manière autonome, permanente et de transporter ainsi le support avec son agrafe en position de pose.

L'extrémité 10 de la fente axiale 11 a une hauteur h (figure 4) inférieure à la hauteur de la partie centrale 4 (7) de l'agrafe lorsque cette dernière est dans son état d'utilisation à température élevée, c'est-à-dire après déformation lorsque sa structure est austénitique. Par exemple la partie centrale 4 de l'agrafe 1 pouvant avoir une forme aplatie à une température inférieure à la température de transition, ses deux côtés s'écartent l'un de l'autre lorsque la température s'élève et viennent alors s'appliquer plus ou moins fermement sur les parois du logement 10, qui exercent alors mécaniquement une action de rétention sur la partie centrale 4 dans sa forme géométrique où sa structure est martensitique. Cette action de rétention contribue, avec celle des crans 18, à maintenir l'agrafe 1 (7) en place dans le corps 8, avec sa partie centrale dans sa forme de hauteur réduite, en un état où elle est prête à sa pose.

Le support 2 comprend des moyens pour extraire l'agrafe 1 (5) du corps 8 et pour la poser sur des éléments osseux 21, 22 de part et d'autre d'une fracture 23 comme représenté à la figure 6. Après extraction du support 2 et enfoncement des branches 6 en position écartée dans les éléments osseux respectifs 21, 22, la libération des contraintes mécaniques de retenue de l'agrafe dans sa forme géométrique initiale entraîne le passage à la structure austénitique, avec corrélativement rapprochement des branches 6 et déformation de la partie centrale 7 (figure 9) d'où un rapprochement des éléments osseux 21, 22.

Le piston 14 est monté coulissant dans la chambre axiale 12 et la fente 11 de manière que son extrémité 25 puisse exercer une poussée longitudinale sur la partie centrale 4 (7) de l'agrafe 1 (5), ce qui permet de pousser l'agrafe hors du corps 8. Sur la tige 26 est ménagé un gradin transversal 27 délimitant d'un côté la partie 13, de forme par exemple cylindrique, et du côté opposé une partie en forme de plaquette 28 se terminant par l'extrémité libre 25. La partie cylindrique 13 se termine par une poignée de préhension manuelle 31 et est dimensionnée pour pouvoir coulisser axialement dans la chambre 12 jusqu'à ce qu'elle puisse venir en butée contre le fond 15 tandis que l'extrémité 25 du piston 14 parvient entre les bords libres 17a des volets 17. La poignée 31 vient alors en même temps en butée contre une bride transversale 30 constituant l'extrémité du corps 8. Avantageusement la face externe de la poignée 31 présente une concavité 32 d'appui du pouce du chirurgien (Figures 3-4).

En position de conditionnement du support 2 et de l'agrafe 1 (5), avant utilisation, la tige 26 du piston 14 est introduite dans la chambre 12 et la fente axiale 11 jusqu'à une position correspondant sensiblement à celle illustrée sur la figure 4. Dans celle-ci l'extrémité libre 25 est proche de la partie centrale 4 (7) de l'agrafe 1 (5) mise en place dans le logement 10 tandis que ses branches 3 (6) sont en appui sur les crans latéraux 18 qui les empêchent de se rapprocher compte tenu de la température ambiante. De même, comme déjà indiqué, les parois du logement 10 exercent sur la partie centrale de l'agrafe une action de contention qui empêche celle-ci de prendre sa forme correspondant à la structure austénitique.

Pour procéder à la pose de l'agrafe, il suffit au chirurgien d'appuyer sur l'extrémité 31 du piston 14, en y plaçant son pouce, tandis que le corps 8 est saisi entre l'index et le majeur de la main qui s'appuient sur la bride 30 (ces doigts étant représentés en traits mixtes à la figure 4). Le pouce exerce sur le piston 14 une poussée axiale jusqu'à ce que l'extrémité libre 25 coulisse au niveau de la fente entre les bords 17a des volets 17, ce qui provoque l'extraction de l'agrafe 1 (7), sa partie centrale étant poussée à l'extérieur du logement 10. Cette poussée provoque une flexion des volets 17 qui s'écartent légèrement et laissent donc sortir l'agrafe. Cette extraction se fait normalement dans la position de pose illustrée à la figure 6, de sorte que le support 2 est également un outil de pose de l'agrafe, ce qui constitue un avantage très appréciable.

Dans la troisième forme de réalisation du support d'agrafe illustrée aux figures 10 et 11, le support 33 comporte un corps 34 formé en deux parties 35, 36 articulées l'une sur l'autre autour d'un axe YY et dont l'une (partie 35) est rabattable sur l'autre.

Dans la première partie 36 est réalisée une fente transversale 37 constituant le logement de la partie centrale de l'agrafe 1 (5) et de part et d'autre de laquelle sont ménagés deux volets flexibles 38 disposés en V ou en forme de toit. Ces volets 38 sont raccordés à leur base à la partie 36 et leurs bords libres 38a se font face en regard de la fente 37. La seconde partie 35 est pourvue d'un doigt 39 formant piston de poussée de la partie centrale 4 (7) de l'agrafe et d'extraction de cette dernière lorsque la seconde partie 35 est rabattue sur la première partie 36, après rotation d'environ 90° pour prendre sa position 35a, 39a illustrée en trait mixte (figure 10).

Dans cette dernière position, le doigt 39 pénètre dans la fente 37, son extrémité se trouvant juste au voisinage de la partie centrale de l'agrafe, prête à exercer une poussée axiale par simple appui du pouce sur la face extérieure de la partie 35, ce qui permet d'extraire et de poser l'agrafe comme dans la réalisation précédente.

Les faces extérieures des parties 35, 36 du corps 34 présentent avantageusement des aspérités telles que des crantages ou moletages 41 facilitant la manipulation par le chirurgien.

Dans les divers modes de réalisation précédemment décrits, le support d'agrafe présente un double avantage par rapport à l'état de la technique antérieure connue : d'une part il permet de maintenir mécaniquement l'agrafe dans sa géométrie correspondant à la structure martensitique de l'alliage constituant cette agrafe, avec ses branches 3 (6) écartées, pratiquement parallèles, et sa partie centrale 4 (7) aplatie, et ce en s'affranchissant de la nécessité de disposer d'un produit cryogénique et d'un conditionnement correspondant. D'autre part le chirurgien n'a plus besoin d'extraire manuellement l'agrafe de son support ou de son conditionnement avant de procéder à sa pose sur les éléments osseux ou les tissus mous, car le support est en même temps l'outil de pose de l'agrafe.

De ce fait, l'agrafe ne risque plus de se déformer intempestivement entre le moment où elle a été extraite de son support et celui où elle peut être effectivement posée, et de devenir inutilisable comme dans l'état de la technique antérieure.

Les deux exemples de réalisation des Fig. 3 à 11 contrairement à l'exemple des Fig. 1 et 2, sont des porte-agrafes ayant la particularité d'être jetables. Ils sont réalisés en un produit plastique peu coûteux stérilisable et biomédical. Le chirurgien peut choisir cette forme de réalisation qui présente l'avantage de supprimer le stockage et le nettoyage en vue d'une nouvelle utilisation.

L'invention n'est pas limitée aux formes de réalisation décrites et peut comporter des variantes d'exécution.

Ainsi par exemple, le support peut être réalisé de manière à exercer une action de contention et de retenue uniquement sur les pattes d'ancrage osseux pour les maintenir écartées, sans exercer une action similaire sur la partie centrale.

## Revendications

1. Support (2;50;33) d'agrafe (1;5) chirurgicale d'ostéosynthèse constituée de deux branches ou pattes (3;6) d'ancrage osseux reliées par une partie centrale (4;7),les branches ou pattes d'ancrage pouvant après sollicitation forcée reprendre automatiquement leur position initiale par un effet élastique, ou superélastique, ou à mémoire de forme du matériau, caractérisé en ce que ce support comporte des moyens mécaniques autonomes (51,53;17;38) adaptés pour maintenir écartées l'une de l'autre de manière permanente les pattes d'ancrage en les bloquant dans leur position d'insertion, tandis que la partie centrale de l'agrafe est maintenue avec un allongement maximum, ces moyens mécaniques pouvant exercer une pression d'écartement sur lesdites pattes, le support pouvant être transporté de manière autonome avec les pattes de l'agrafe écartées.

2. Support selon la revendication 1, caractérisé en ce que lesdits moyens mécaniques sont en outre conformés pour pouvoir se refermer sur la partie centrale (4;7) de l'agrafe (3;5) lorsque cette partie est sous une forme géométrique adaptée à sa pose chirurgicale.

3. Support selon la revendication 2, caractérisé en ce que lesdits moyens comprennent deux éléments allongés (51) formant mâchoires, dans chacun desquels est ménagée une empreinte (52) conjuguée de la forme de la partie centrale (4;7) de l'agrafe (1;5) afin que ladite partie centrale puisse être emprisonnée par les deux mâchoires appliquées l'une sur l'autre, avec leurs empreintes en vis-à-vis, en ce que les faces d'extrémité (51a) de ces mâchoires sont conformées pour former des butées de retenue des pattes (3;6) en position d'écartement, et en ce que ce support comprend une pince (53) d'écartement ou de rapprochement des mâchoires, aux branches (54) de laquelle ces dernières sont fixées.

4. Support selon la revendication 3, caractérisé en ce que la longueur des mâchoires (51) est au moins légèrement supérieure à la longueur de la partie centrale (7) de l'agrafe (5) lorsque ladite partie centrale est rectiligne, ou à la longueur totale déployée de cette partie centrale lorsque celle-ci présente une ondulation ou tout autre forme autorisant un allongement, telle qu'un oeillet (4) ou boutonnière.

5. Support selon la revendication 3 ou 4, caractérisé en ce qu'il est en un matériau réutilisable.

6. Support selon la revendication 1, caractérisé en ce que lesdits moyens comprennent un corps (8) présentant à une extrémité un logement (10) de réception de la partie centrale de l'agrafe, et des volets flexibles (17) de retenue des branches de l'agrafe en position écartée, et en ce que des moyens (14) sont prévus pour extraire l'agrafe du corps et pour la poser.

7. Support selon la revendication 6, caractérisé en ce que le corps (8) a une forme sensiblement tubulaire dans laquelle est ménagé longitudinalement un évidement (11, 12) comportant une fente (11) dont une extrémité constitue le logement (10) de la partie centrale (4; 7) de l'agrafe (1; 5) de part et d'autre duquel s'articulent sur le corps deux volets flexibles (17) disposés en V et dont les bords libres (17a) se font face au niveau de la fente (11) et des branches (3; 6) de l'agrafe, et en ce que des crans (18) sont agencés sur les extrémités des volets de manière à former des butées de retenue et de maintien des branches de l'agrafe en position écartée de manière permanente et autonome, tant que les volets flexibles ne sont pas écartés l'un de l'autre.

8. Support selon la revendication 7, destiné particulièrement aux agrafes (1) à mémoire de forme, caractérisé en ce que le logement (10) de la partie centrale (4) de l'agrafe (1) a une hauteur (h) inférieure à la hauteur de la partie centrale de l'agrafe lorsque cette dernière est dans son état d'utilisation à température élevée, de telle sorte que les parois dudit logement peuvent maintenir mécaniquement ladite partie centrale en place dans sa forme de hauteur réduite, correspondant à son état de non utilisation à température basse, en réalisant ainsi une contention de l'agrafe.

9. Support selon la revendication 7 ou 8, caractérisé en ce que les moyens pour extraire l'agrafe (1; 5) consistent en un piston (14) monté coulissant dans l'évidement longitudinal (11, 12) de manière que son extrémité (25) puisse exercer une poussée longitudinale sur la partie centrale (4; 7) de l'agrafe.

10. Support selon la revendication 9, caractérisé en ce que le piston (14) comporte une tige (26) sur laquelle est ménagé un gradin transversal (27) pouvant venir en butée contre un épaulement correspondant (15) formant le fond d'une chambre (12) de l'évidement du corps, afin de limiter la course du piston.

11. Support selon la revendication 10, caractérisé en ce que le piston (14) est pourvu d'une poignée terminale (31) dans laquelle est ménagée une concavité (32) facilitant sa préhension manuelle.

12. Support selon la revendication 11, caractérisé en ce que la tige (26) du piston (14) est constituée d'une partie (13) attenante à la poignée (31), et d'une plaquette (28), ladite partie (13), par exemple cylindrique, pouvant coulisser dans la chambre (12) du corps (8), tandis que la plaquette (28) coulisse axialement dans une fente (11) du corps se terminant par le logement (10).

13. Support (33) selon la revendication 1, caractérisé en ce que le corps (34) est formé en deux parties (35, 36) articulées l'une sur l'autre et dont l'une (35) est rabattable sur l'autre, en ce que dans une partie (36) est réalisée une fente transversale (37) constituant le logement de la partie centrale (4; 7) de l'agrafe (1; 5) et de part et d'autre de laquelle sont ménagés en V deux volets flexibles (38) dont les bords libres (38a) se font face en regard de ladite fente, et en ce que l'autre partie (36) est pourvue d'un doigt (39) formant piston de poussée de la partie centrale et d'extraction de l'agrafe lorsque cette seconde partie est rabattue sur la première, le doigt (39) pénétrant alors dans la fente (37) et entre les volets (38).

14. Support selon les revendications 1 et 7 à 12, caractérisé en ce qu'il est en un matériau jetable.

15. Support selon la revendication 3 ou 4, caractérisé en ce que les branches (54) de la pince (53) sont munies de crans (60,61) pouvant engrener ensemble dans une position réglable lorsque la pince est refermée et que les mâchoires (51) sont appliquées l'une sur l'autre, de telle sorte que le support (50) peut alors être transporté de manière autonome avec les pattes (6) de l'agrafe (5) maintenues écartées.
